(19) Europäisches Patentamt
European Patent Office
Office européen des brevets

(11) Veröffentlichungsnummer: **0 027 897**
**A1**

(12) **EUROPÄISCHE PATENTANMELDUNG**

(21) Anmeldenummer: **80105767.0**

(22) Anmeldetag: **25.09.80**

(51) Int. Cl.³: **C 07 D 487/14,** D 06 L 3/12
// (C07D487/14, 243/00, 235/00, 235/00),(C07D487/14, 241/00, 235/00, 235/00)

(30) Priorität: **02.10.79 DE 2939916**

(43) Veröffentlichungstag der Anmeldung: **06.05.81**
**Patentblatt 81/18**

(84) Benannte Vertragsstaaten: **CH DE FR GB IT LI NL**

(71) Anmelder: **HOECHST AKTIENGESELLSCHAFT, Zentrale Patentabteilung Postfach 80 03 20, D-6230 Frankfurt/Main 80 (DE)**

(72) Erfinder: **Schönberger, Norbert, Dr., Tilsiter Weg 3, D-6343 Wehrheim (DE)**
Erfinder: **Schinzel, Erich, Dr., Ostpreussenstrasse 43, D-6238 Hofheim am Taunus (DE)**
Erfinder: **Martini, Thomas, Dr., Am Schellberg 42, D-6232 Bad Soden am Taunus (DE)**
Erfinder: **Rösch, Günter, Hohlweg 17, D-6232 Bad Soden am Taunus (DE)**

(54) **Quaternierte, verbrückte Benzimidazolyl-benzimidazole, Verfahren zu deren Herstellung und deren Verwendung.**

(57) Verbindungen der allgemeinen Formel (I)

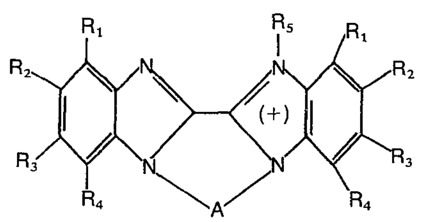

$X^{(-)}$ (1)

wobei

$R_1$ Wasserstoff, Halogen, Alkyl, Alkoxy oder zusammen mit $R_2$ einen ankondensierten Benzoring,

$R_2$ Wasserstoff, Alkyl, Alkoxy, Halogen, Phenyl, Cyan, Carboalkoxy, Carboxy, Carbonamido, Mono- oder Di-alkylcarbonamido, Sulfo, Alkylsulfonyl, Alkyloxysulfonyl, Sulfonamido, Mono- oder Di-alkylsulfonamido oder zusammen mit $R_3$ einen ankondensierten Benzoring,

$R_3$ die gleiche Bedeutung hat wie $R_2$,

$R_4$ Wasserstoff, Alkyl, Alkoxy, Halogen oder zusammen mit $R_3$ einen ankondensierten Benzoring,

$R_5$ Alkyl, Hydroxyalkyl, Alkoxyalkyl, gegebenenfalls substituiertes Aralkyl, Cyanäthyl, Cycloalkyl oder eine Gruppe der Formeln

$-CH_2CN$, $-CH_2CONH_2$ oder $-CH_2COO$ Alkyl

A eine Gruppe der Formeln

$$\left[\begin{array}{c}R_6\\C\\R_6\end{array}\right]_n \quad \text{oder} \quad -(CH_2)_4-$$

$R_6$ Wasserstoff, Alkyl, Hydroxyalkyl, Alkoxyalkyl, Aralkyl oder gegebenenfalls substituiertes Phenyl,

n 1, 2 oder 3 und

$X^{(-)}$ ein Halogenid-, Alkylsulfonat-, Alkylsulfat-, Alkylphenylsulfonat- oder Phenylsulfonat-ion bedeutet, sowie Verfahren zu deren Herstellung und deren Verwendung als optische Aufheller.

EP 0 027 897 A1

0027897

HOECHST AKTIENGESELLSCHAFT HOE 79/F 262 Dr.OT/jk

# Quaternierte, verbrückte Benzimidazolyl-benzimidazole, Verfahren zu deren Herstellung und deren Verwendung

Gegenstand der Erfindung sind neue quaternierte, verbrückte Benzimidazolyl-benzimidazole der allgemeinen Formel (1)

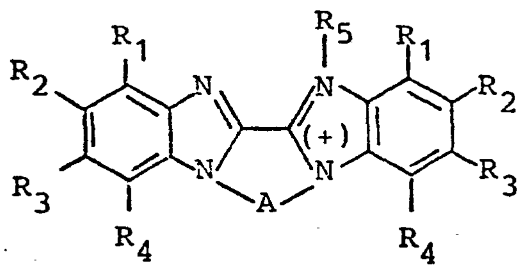

$X^{(-)}$ (1)

wobei $R_1$ Wasserstoff, Halogen, Alkyl, Alkoxy oder zusammen mit $R_2$ einen ankondensierten Benzoring,
$R_2$ Wasserstoff, Alkyl, Alkoxy, Halogen, Phenyl, Cyan, Carboalkoxy, Carboxy, Carbonamido, Mono- oder Di-alkylcarbonamido, Sulfo, Alkylsulfonyl, Alkyloxysulfonyl, Sulfonamido, Mono- oder Di-alkylsulfonamido oder zusammen mit $R_3$ einen ankondensierten Benzoring,
$R_3$ die gleiche Bedeutung hat wie $R_2$,
$R_4$ Wasserstoff, Alkyl, Alkoxy, Halogen oder zusammen mit $R_3$ einen ankondensierten Benzoring,
$R_5$ Alkyl, Hydroxyalkyl, Alkoxyalkyl, gegebenenfalls substituiertes Aralkyl, Cyanäthyl, Cycloalkyl oder eine Gruppe der Formeln
$-CH_2CN-$, $-CH_2CONH_2$ oder $-CH_2COO$-Alkyl,
A eine Gruppe der Formeln

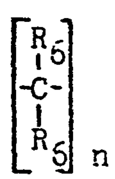 oder $-(CH_2)_4-$

$R_6$ Wasserstoff, Alkyl, Hydroxyalkyl, Alkoxyalkyl, Aralkyl oder gegebenenfalls substituiertes Phenyl,

n 1,2 oder 3 und
$X^{(-)}$ ein Halogenid-, Alkylsulfonat-, Alkylsulfat-, Alkylphenylsulfonat- oder Phenylsulfonat-ion bedeutet.

Bevorzugt sind solche Verbindungen der Formel 1, worin
$R_1$ Wasserstoff, Alkyl oder zusammen mit $R_2$ einen ankondensierten Benzoring,
$R_2$ Wasserstoff, Alkyl, Alkoxy, Halogen, Cyan, Carboalkoxy, Dialkylcarbonamido, Sulfo, Alkylsulfonyl, Alkyloxysulfonyl, Sulfonamido oder zusammen mit $R_3$ einen ankondensierten Benzoring,
$R_3$ die gleiche Bedeutung wie $R_2$ hat,
$R_4$ Wasserstoff, Alkyl oder zusammen mit $R_3$ einen ankondensierten Benzoring,
$R_5$ Alkyl, Hydroxyalkyl, Alkoxyalkyl, gegebenenfalls substituiertes Aralkyl, Cyanäthyl oder eine Gruppe der Formeln
$-CH_2CN$, $-CH_2CONH_2$, $-CH_2COO$-Alkyl,
A eine Gruppe der Formeln

$$\left[ -\overset{R_6}{\underset{R_6}{\overset{|}{\underset{|}{C}}}}- \right]_n \quad \text{oder} \quad -(CH_2)_4-$$

$R_6$ Wasserstoff, Alkyl, Hydroxyalkyl, Alkoxyalkyl, Aralkyl oder gegebenenfalls substituiertes Phenyl,
n 1, 2 oder 3 und $X^{(-)}$ ein Halogenid-, Alkylsulfonat-, Alkylsulfat-, Alkylphenylsulfonat- oder Phenylsulfonat-ion bedeutet.

Besonders bevorzugt sind solche Verbindungen der Formel (1), worin $R_1$ und $R_4$ Wasserstoff, Methyl, Ethyl, Methoxy, Chlor oder
$R_1$ zusammen mit $R_2$ bzw. $R_3$ zusammen mit $R_4$ einen ankondensierten Benzoring,

$R_2$ und $R_3$ Wasserstoff, Methyl, Ethyl, Chlor, Alkylsulfonyl, Cyan, Carboxy, Carboalkoxy oder Carbonamido, $R_5$ Alkyl, Hydroxyalkyl, gegebenenfalls durch Chlor, Methyl oder Methoxy substituiertes Benzyl oder eine Gruppe der Formeln $-CH_2CN$, $-CH_2CONH_2$, oder $-CH_2COO$-Alkyl, A Methylen, Ethylen, Trimethylen oder Tetramethylen, die jeweils durch Alkyl, Alkoxy, Phenyl, Cyanphenyl oder Chlorphenyl substituiert sein können und $X^{(-)}$ ein Chlorid-, Ethosulfat-, Methosulfat-, Methylsulfonat-, Tolylsulfonat- oder Phenylsulfonat-ion bedeutet.

Besonders bevorzugt sind auch die Verbindungen der Formel (1), worin $R_1$ und $R_4$ Wasserstoff, $R_2$ Wasserstoff, Alkyl, Alkoxy oder Chlor, $R_3$ Wasserstoff, Alkyl, Alkoxy, Chlor, Methylsulfonyl, Cyan oder Carboalkoxy, $R_5$ Methyl, A Methylen, Ethylen oder Propylen und $X^{(-)}$ Chlorid, Methosulfat, Ethosulfat Methylsulfonat oder p-Tolylsulfonat bedeutet.

Besonders bevorzugt sind auch die Verbindungen der Formel (1), worin $R_1$ und $R_4$ Wasserstoff, $R_2$ Wasserstoff, Alkyl, Alkoxy oder Chlor, $R_3$ Wasserstoff, wenn $R_2$ ungleich Wasserstoff ist, sowie Alkyl, Chlor, Methoxy, Ethoxy, Methylsulfonyl, Cyano, Carboalkoxy, $R_5$ Methyl, A Ethylen und $X^{(-)}$ Chlorid, Methosulfat, Methylsulfonat oder Tolylsulfonat bedeutet.

Die oben angegebenen Alkyl- und Alkoxygruppen sowie andere, daraus abgeleitete Gruppen enthalten jeweils 1 bis 4 C-Atome. Cycloalkyl bedeutet vorzugsweise Cyclohexyl und Aralkyl ist vorzugsweise Benzyl. Die Aralkyl- und auch die Phenylgruppe kann gegebenenfalls durch Halogen, Alkyl oder

Cyan substituiert sein. Die Substituenten in den beiden Benzimidazolringen können jeweils gleich oder verschieden sein.

Die Herstellung der Verbindungen der Formel (1) erfolgt in bekannter Weise durch Alkylierung einer Verbindung der Formel (2)

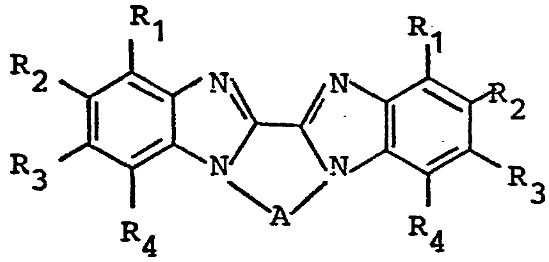

(2)

mit einer Verbindung der Formel

$$X - R_5.$$

Diese Alkylierung erfolgt vorzugsweise in einem inerten organischen Lösungsmittel, insbesondere in einem aprotisch polaren Lösungsmittel wie Dimethylformamid bei Temperaturen von ca. 50 bis 150°C.

Ein weiteres Herstellverfahren besteht darin, daß man eine Verbindung der Formel (3)

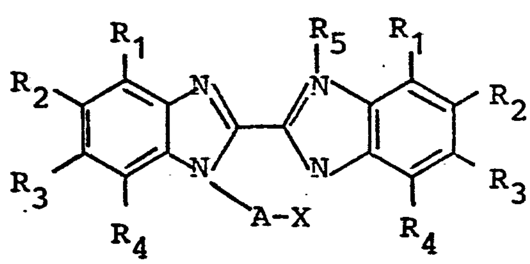

(3)

durch Erhitzen auf ca. 50 bis 150°C in einem inerten organischen Lösungsmittel, gegebenenfalls unter Zusatz eines Katalysators, cyclisiert. Man kann die Verbindungen der Formel (1) aber auch in der Weise herstellen, daß man eine Verbindung der Formel (4)

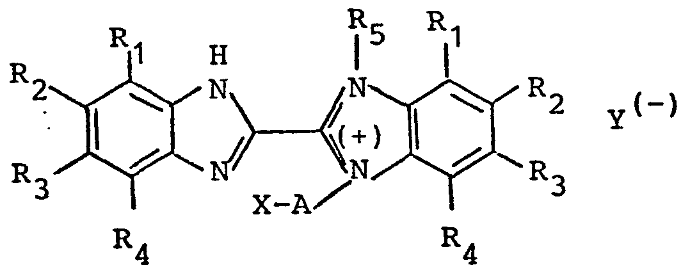

(4)

wobei $Y^{(-)}$ ein farbloses Anion bedeutet, durch Erhitzen auf ca. 50 bis 150°C in einem inerten organischen Lösungsmittel, gegebenenfalls in Gegenwart einer basischen Verbindung, cyclisiert. Die Herstellung der Verbindungen der Formel (1) gelingt auch durch oxydative Kupplung in 2,2'-Stellung von Verbindungen der Formel (5)

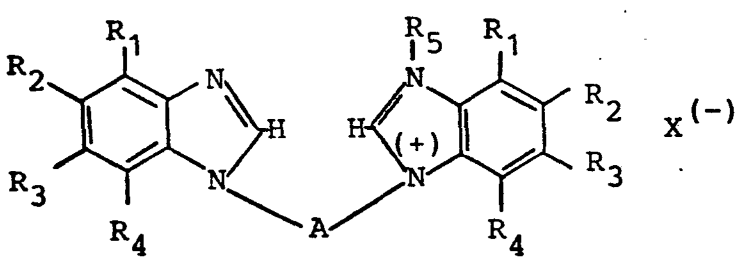

(5)

Die als Ausgangsverbindungen dienenden Benzimidazole der Formel (2) werden erhalten, indem man Bis-benzimidazole der Formel (6)

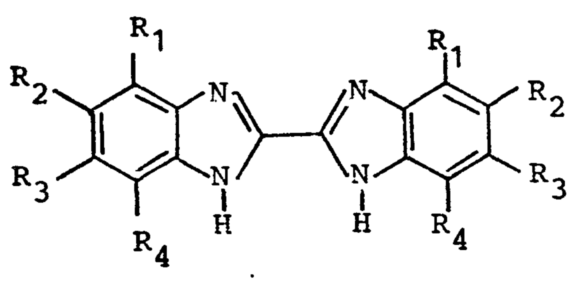

(6)

mit einer Verbindung Z-A-Z umsetzt, wobei Z ein Halogenatom darstellt. Beispiele hierfür sind Methylenhalogenide, 1,2-Dibromäthan, Trimethylenbromid oder 1,4-Dibrombutan. Die Zugabe einer basischen Verbindung ist bei dieser Reaktion von Vorteil. Die Einfügung der Brücke A

kann aber auch durch Umsetzung der Verbindungen der Formel (6) mit Alkylenoxid erfolgen.

Eine andere Möglichkeit der Herstellung der Bis-benzimidazole der Formel (2) besteht darin, daß man eine Verbindung der Formel (7)

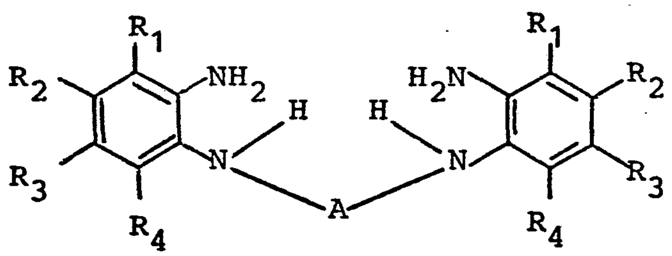

(7)

mit Oxalsäure oder einem Oxalsäurederivat wie etwa Oxalsäurediamid, Dicyan, Oxalsäurediester oder Oxalylchlorid umsetzt.

Ferner lassen sich die Bis-benzimidazole der Formel (2) herstellen durch oxydative Verknüpfung von Verbindungen der Formel (8)

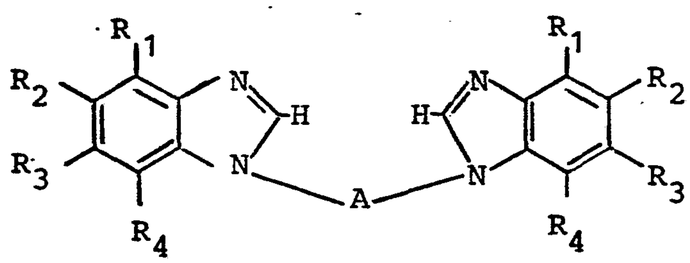

(8)

in 2,2'-Stellung nach bekannten Verfahren (B. A. Tertov u.a., TETRAHEDRON LETTERS 1968, 4445).

Bedingt durch die Herstellverfahren können die überbrückten Bis-benz-imidazole der Formel (2) und dementsprechend auch die erfindungsgemäßen Verbindungen (1) in der Theorie sowohl in der symmetrischen als auch in der unsymmetrischen Form anfallen, d. h. der Substituent $R_1$ in dem

einen Benzimidazolring kann identisch sein entweder mit dem Substituenten $R_1$ oder mit $R_4$ im anderen Benzimidazolring. Entsprechend kann der eine Substituent $R_2$ identisch sein mit dem anderen Substituenten $R_2$ oder aber mit $R_3$. Damit ergibt sich rein theoretisch die Möglichkeit, daß Gemische aus der symmetrischen und der unsymmetrischen Verbindung entstehen können. In der Praxis konnten jedoch für eine solche Annahme keine Anhaltspunkte ausgemacht werden, da sich sämtliche hergestellten Verbindungen der Formel (1) als chromatographisch einheitlich erwiesen.

Die Bis-benzimidazole der Formel (6) kann man analog zu dem von S. Hünig. Ann. 765(1972), 126 beschriebenen Verfahren aus Phenylendiaminen und Oxalsäurederivaten, wie etwa Oxalsäurediamid, herstellen oder man setzt Oxalylchlorid mit o-Nitro-aminobenzolen um, reduziert anschließend die Nitrogruppe und schließt die Imidazolringe analog zu üblichen Verfahren. Eine andere Möglichkeit zum Herstellen von vor allem unsymmetrisch substituierten Bisbenzimidazolen der Formel (6) besteht in der Reaktion eines Derivates der Benzimidazolyl-(2)-carbonsäure, wie z. B. des Säurechlorids, mit geeigneten Phenylendiaminen oder o-Nitroamino-benzolen. Vor dem Ringschluß wird dann die Nitrogruppe zur Aminogruppe reduziert.

Die neuen Verbindungen dienen zum Aufhellen von organischem Material, wie etwa Baumwolle, vor allem von synthetischen Fasern, z. B. aus Polyestern wie Polyterephthalsäureglykolestern, Polyamiden wie Polymeren auf Basis von Hexamethylendiaminadipat oder Caprolactam, Celluloseestern wie Cellulose-2 1/2-Acetat und Cellulosetriacetat und insbesondere aus Polyacrylnitril.

Man kann das organische Material beispielsweise dadurch

aufhellen, daß man diesem geringe Mengen erfindungsgemäßer optischer Aufheller, zweckmäßig 0,001 bis 1%, bezogen auf das aufzuhellende Material, gegebenenfalls zusammen mit anderen Substanzen, wie Weichmachern, Stabilisatoren oder Pigmenten, einverleibt. Man kann die Aufheller beispielsweise gelöst in Weichmachern, wie Dioctylphthalat, oder zusammen mit Stabilisatoren, wie Dibutylzinndilaurat oder Natrium-pentaoctyl-tripolyphosphat, oder zusammen mit Pigmenten, beispielsweise Titandioxid, in die Kunststoffe einarbeiten. Je nach der Art des aufzuhellenden Materials kann der Aufheller auch in den Monomeren vor der Polymerisation, in der Polymerenmasse oder zusammen mit den Polymeren in einem Lösungsmittel gelöst werden.

Das so vorbehandelte Material wird hierauf nach an sich bekannten Verfahren, wie Verspinnen und Strecken, in die gewünschte endgültige Form gebracht. Man kann die Aufheller auch in Appreturen einarbeiten, beispielsweise in Appreturen für Textilfasern wie Polyvinylalkohol, oder in Harze bzw. Harzvorkondensate wie z. B. Methylolverbindungen von Ethylenharnstoff, die zur Textilbehandlung dienen.

Die erfindungsgemäßen Verbindungen eignen sich auch zum Aufhellen von Papier im Oberflächenstrich. Vorzugsweise wird jedoch farbloses, hochmolekulares organisches Material in Form von Fasern aufgehellt. Zum Aufhellen dieser Fasermaterialien verwendet man vorteilhaft eine wäßrige Lösung oder Dispersion erfindungsgemäßer Benzimidazole der Formel (1). Die Aufhellerdispersion bzw. Lösung weist hierbei vorzugsweise einen Gehalt von 0,005 bis 0,5 % an erfindungsgemäßem Benzimidazol, bezogen auf das Fasermaterial, auf. Daneben kann die Dispersion bzw. Lösung Hilfsstoffe enthalten, wie Dispergatoren, beispielsweise Kondensationsprodukte, 10 bis 18 Kohlenstoffatome aufweisender Fettalkohole oder Alkylphenole mit 15 bis 25 Mol

Ethylenoxyd, oder Kondensationsprodukte 16 bis 18 Kohlenstoffatome aufweisender Alkylmono- oder Polyamine mit mindestens 10 Mol Ethylenoxyd, organische Säuren wie Ameisen-, Oxal- oder Essigsäure, Waschmittel, Quellmittel wie Di- oder Trichlorbenzole, Netzmittel wie Sulfobernsteinsäure-alkylester, Bleichmittel wie Natriumchlorit, Peroxide oder Hydrosulfite, sowie gegebenenfalls Aufhellungsmittel der gleichen oder anderer Klassen, wie z. B. celluloseaffine Derivate des Stilbens.

Die Aufhellung des Fasermaterials mit der wäßrigen Aufhellerflotte erfolgt entweder im Ausziehverfahren bei Temperaturen von vorzugsweie 30 bis 150°C oder im Foulardverfahren. Im letzteren Falle imprägniert man die Ware mit einer beispielsweise 0,2 bis 0,5%igen Aufhellerdispersion und stellt das Färbegut z. B. durch trockene oder feuchte Hitzebehandlung fertig, beispielsweise durch Dämpfen bei 2 Atmosphären oder nach erfolgter Trocknung durch kurzes trockenes Erhitzen auf 180 bis 220°C, wobei gegebenenfalls das Gewebe zugleich thermofixiert wird. Das derart behandelte Fasermaterial wird zum Schluß gespült und getrocknet.

Erfindungsgemäß optisch aufgehelltes, farbloses, hochmolekulares, organisches Material, insbesondere das nach dem Ausziehverfahren aufgehellte natürliche oder synthetische Fasermaterial, weist ein gefälliges, rein weißes, blauviolett bis blaustichig fluoreszierendes Aussehen auf, in hellen Farbtönen gefärbtes und erfindungsgemäß weißgetöntes derartiges Material zeichnet sich durch reinen Farbton aus.

Waschflotten, die Benzimidazole der Formel (1) enthalten, verleihen beim Waschen den damit behandelten Textilfasern, beispielsweise synthetischen Polyamid-, Polyester- und Celluloseesterfasern, insbesondere aber Polyacrylnitrilfasern, einen brillanten Aspekt im Tageslicht.

BEISPIEL 1:

Zum Herstellen der quartären Verbindung der Formel

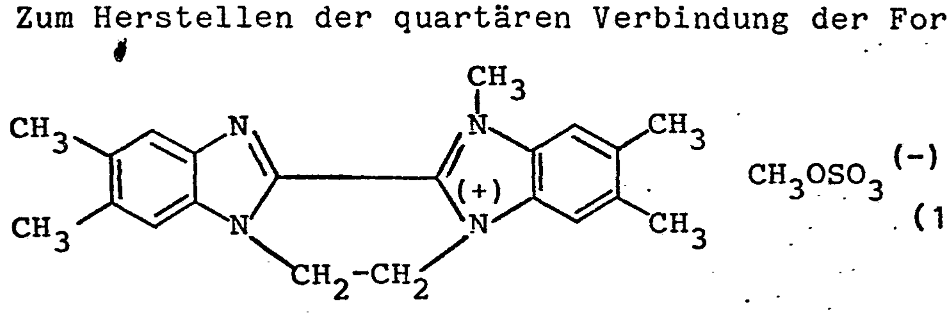

(101)

werden 31,6 g 1,1'-Dimethylen-2,2'-bi(5,5',6,6'-tetramethyl)-benzimidazol in 300 ml Dimethylformamid suspendiert. Nach Zugabe von 10 ml Dimethylsulfat wird 1 Stunde auf 80°C, dann 10 Minuten auf 120°C unter Rühren erhitzt, wobei eine klare Lösung entsteht. Man läßt 1 Stunde bei 80°C nachrühren, kühlt auf 5°C ab und isoliert das ausgefallene Reaktionsprodukt durch Filtration. Der lösungsmittelhaltige Filterkuchen wird mit Toluol gewaschen und anschließend im Vakuum bei 60°C getrocknet. Rohausbeute: 42,0 g, entsprechend 95 % der Theorie. Umkristallisiert aus Methanol schmilzt die leicht gelbliche Verbindung bei 346 - 350°C unter Zersetzung.

Die Verbindung löst sich sowohl in Wasser als auch in Dimethylformamid mit blauvioletter Fluoreszenz am Tageslicht und eignet sich vorzüglich zum Aufhellen von organischen Materialien, insbesondere von Polyacrylnitrilfasern.

Das als Ausgangsprodukt verwendete 1,1'-Dimethylen-2,2'-bi(5,5',6,6'-tetramethyl)-benzimidazol wird wir folgt hergestellt:

11,6 g 2,2'-Bi(5,5',6,6'-tetramethyl)-benzimidazol werden zusammen mit 13,8 g Kaliumkarbonat in 200 ml Dimethylformamid suspendiert. Nach Zugabe von 4,3 ml 1,2-Dibromethan wird 8 Stunden bei 80°C, dann 6 Stunden bei 100°C

gerührt. Man läßt auf Raumtemperatur abkühlen, filtriert und wäscht den Filterkuchen erst zweimal mit DMF, anschließend mit Wasser. Rohausbeute nach Trocknen bei 60°C im Vakuum: 10,1 g. Die Verbindung kann aus Eisessig umkristallisiert werden und zeigt dann korrekte Werte der Elementaranalyse. Schmelzpunkt: 360°C.

Das als Ausgangsprodukt verwendete 2,2'-Bi(5,5',6,6'-tetramethyl)-benzimidazol wird wie folgt hergestellt: 40,8 g 4,5-Dimethyl-1,2-diaminobenzol werden zusammen mit 13,2 g Oxalsäurediamid in 100 ml Glykol suspendiert. Man heizt auf 180°C und rührt bei dieser Temperatur 40 Std. im leichten Stickstoffstrom, wobei etwas Wasser abdestilliert. Danach läßt man abkühlen, verdünnt mit 100 ml Ethanol, filtriert und wäscht den Filterkuchen mit Ethanol. Nach Trocknen im Vakuum erhält man 31,2 g 2,2'-Bi(5,5',6,6'-tetramethyl)-benzimidazol, das durch Umkristallisation aus Dimethylformamid weiter gereinigt werden kann. Schmp. 360°C.

BEISPIEL 2:

Zur Herstellung der quartären Verbindung der Formel

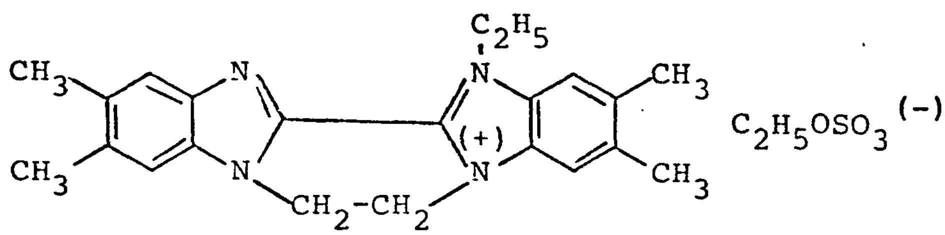

(102)

werden 31,6 g 1,1'-Dimethylen-2,2'-bi(5,5',6,6'-tetramethyl)-benzimidazol in 300 ml Dimethylformamid suspendiert. Nach Zugabe von 12 ml Diethylsulfat wird 2 Stunden auf 80°C unter Rühren erhitzt, wobei eine klare Lösung entsteht. Man kühlt auf Raumteperatur ab und isoliert das auskristallisierte Produkt durch Filtration. Der Filterkuchen wird zweimal mit wenig Methanol gewaschen und anschließend im Vakuum bei 60°C getrocknet.
Rohausbeute: 40,0 g, entsprechend 85% der Theorie.
Die Verbindung löst sich sowohl in Wasser als auch in Dimethylformamid am Tageslicht mit blauvioletter Fluoreszenz.

BEISPIEL 3:

Die quartäre Verbindung der Formel

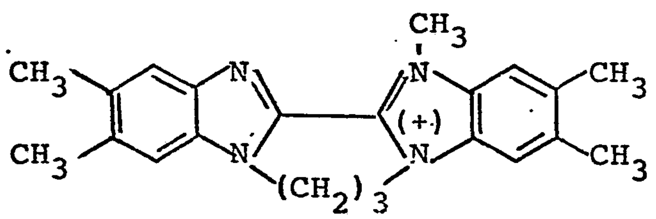 $CH_3OSO_3^-$ (103)

wird wie folgt hergestellt:
3,3 g 1,1'-Trimethylen-2,2'-bi(5,5',6,6'-tetramethyl)-benzimidazol werden in 35 ml Dimethylformamid suspendiert. Nach dem Erwärmen auf 100°C wird 1 ml Dimethylsulfat zugesetzt und 2 Stunden bei 95 - 100°C gerührt. Es bildet sich eine klare, gelbliche Lösung, aus welcher beim Erkalten auf Raumtemperatur das quartäre Reaktionsprodukt auskristallisiert. Man saugt ab, wäscht den Filterkuchen mit Toluol und trocknet bei 60°C im Vakuum. Rohausbeute: 3,6 g, entsprechend 79 % der Theorie. In stark verdünnter Lösung in Wasser oder Dimethylformamid zeigt die schwach gelb gefärbte Verbindung am Tageslicht eine rotstichig-blaue Fluoreszenz.

Das als Ausgangsprodukt verwendete 1,1'-Trimethylen-2,2'-bi(5,5',6,6'-tetramethyl)-benzimidazol wird wie folgt erhalten:
14,5 g 2,2'-Bi-(5,5',6,6'-tetramethyl)benzimidazol werden zusammen mit 16,6g Kaliumkarbonat in 250 ml Dimethylformamid suspendiert. Nach Zugabe von 12,6 g 1,3-Dibrompropan wird 8 Stunden bei 80 bis 85°C und anschließend noch 19 Stunden bei 100°C gerührt. Man kühlt auf Raumtemperatur ab, saugt ab, wäscht mit Dimethylformamid und anschließend mit Wasser neutral und trocknet bei 60°C im Vakuum. Rohausbeute: 5,1 g entsprechend 31 % der Theorie. Die Verbindung wird aus Dimethylformamid umgelöst und zeigt dann korrekte Werte der Elementaranalyse. Schmelzpunkt: 360°C.

Die Herstellung des als Ausgangsprodukt verwendeten 2,2'-Bi(5,5',6,6'-tetramethyl)-benzimidazols ist in Beispiel 1 beschrieben.

BEISPIEL 4:

Die quartäre Verbindung der Formel

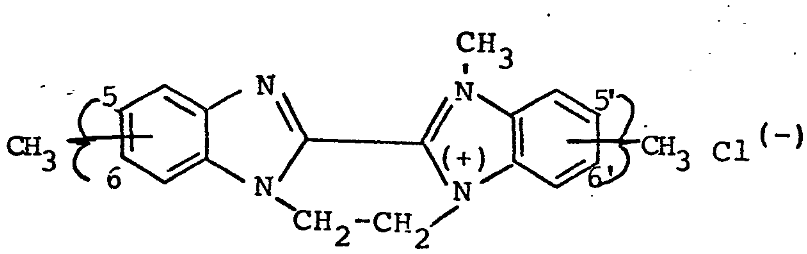

(104)

wird in folgender Weise erhalten:
20 g 1,1'-Dimethylen-2,2'-bi(5 oder 6, 5' oder 6'-dimethyl)-benzimidazol werden in 200 ml Dimethylformamid suspendiert und bei 100°C mit 7 ml Dimethylsulfat versetzt. Es bildet sich eine klare Lösung, die bei 95-100°C 12 Stunden nachgerührt wird. Nach dem Erkalten verdünnt man mit 500 ml Wasser, filtriert und klärt das Filtrat nach Verrühren mit Kohle. Durch Sättigung mit Kochsalz scheidet man die quartäre Verbidung ab, die abgenutscht und mit gesättigter Natriumchlorid-Lösung gewaschen wird. Man erhält nach dem Trocknen 18 g einer gelblichen, in heißem Wasser klar löslichen Verbindung, die neben Kochsalz und Wasser 69 % des quartären Produktes (104) enthält.

Das als Ausgangsprodukt verwendete 1,1'-Dimethylen-2,2'-bi-(5 oder 6, 5' oder 6'-dimethyl)-benzimidazol wird wie folgt hergestellt:
13,1 g 2,2'-Bi(5 oder 6, 5' oder 6'-dimethyl)-benzimidazol werden zusammen mit 17,3 g Kaliumcarbonat in 150 ml Dimethylformamid suspendiert. Nach Zugabe von 5,4 ml 1,2-Dibromethan wird 5 Stunden bei 100°C verrührt. Man läßt auf Raumtemperatur abkühlen, saugt ab und wäscht den Filterkuchen zweimal mit Dimethylformamid und anschließend mit

Wasser neutral. Rohausbeute nach Trocknen bei 60°C im Vakuum: 10,6 g. Die Verbindung kann aus Ethylglykol umkristallisiert werden und zeigt dann korrekte Werte der Elementaranalyse. Schmelzpunkt: 360°C.

Das als Ausgangsprodukt verwendete 2,2'-Bi(5 oder 6, 5' oder 6'-dimethyl)-benzimidazol wird wie folgt hergestellt: 122,2 g 3,4-Diaminotoluol und 44 g Oxalsäurediamid werden in 300 ml Glykol suspendiert. Man heizt auf eine Innentemperatur von 195 - 200°C und rührt bei dieser Temperatur 45 Stunden im leichten Stickstoffstrom nach. Man läßt auf Raumtemperatur erkalten, verdünnt mit 300 ml Methanol, saugt ab und wäscht den Filterkuchen mit Methanol. Nach dem Trocknen im Vakuum erhält man 70,5 g 2,2'-Bi(5 oder 6, 5' oder 6'-dimethyl)-benzimidazol, das durch Umlösung aus Dimethylformamid gereinigt werden kann.
Schmelzpunkt: 360°C.

Bedingt durch diesen Reaktionsweg sind die Positionen 5 und 6 bzw. 5' und 6' als gleichwertig anzusehen und es kann nicht angegeben werden ob sich die Methylgruppe in 5- bzw. 5'-Stellung oder in 6- bzw. 6'-Stellung befindet. Es wurde daher die obige Darstellungsweise mit der geschweiften Klammer für die Endverbindung gewählt.

BEISPIEL 5:

Zur Synthese der quartären Verbindung

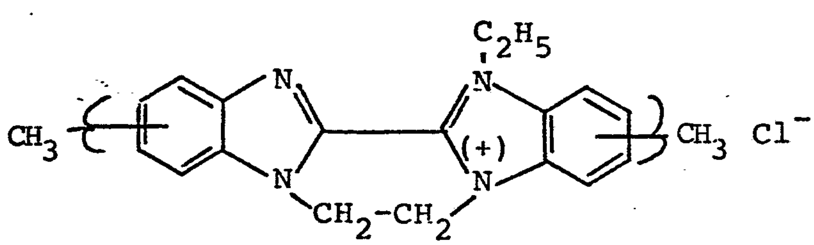

(105)

verfährt man wie folgt:
2,88 g des 1,1'-Dimethylen-2,2'-bi-(5 oder 6, 5' oder 6'-dimethyl)-benzimidazols werden in 50 ml Dimethylformamid suspendiert und bei 80°C mit 1,4 ml Diethylsulfat versetzt. Man rührt 5 Stunden bei 80°C nach, läßt auf Raumtemperatur erkalten und gibt das Reaktionsgemisch auf die vierfache Menge Wasser. Von geringen, unlöslichen Verunreinigungen wird abfiltriert und das Filtrat mit ca. 20 g Natriumchlorid versetzt. Das auskristallisierte Produkt wird abgesaugt, mit gesättigter Kochsalzlösung gewaschen und bei 60°C im Vakuum getrocknet. Man erhält 2,3 g einer fast farblosen, in heißem Wasser klar löslichen Verbindung, die neben Kochsalz und Wasser 60 % des quartären Produktes (105) enthält.

Die Herstellung des als Ausgangmaterial verwendeten 1,1'-Dimethylen-2,2'-bi-(5 oder 6, 5' oder 6'-dimethyl)-benzimidazols ist im vorstehenden Beispiel beschrieben.

BEISPIEL 6:

Zur Herstellung der quartären Verbindung der Formel

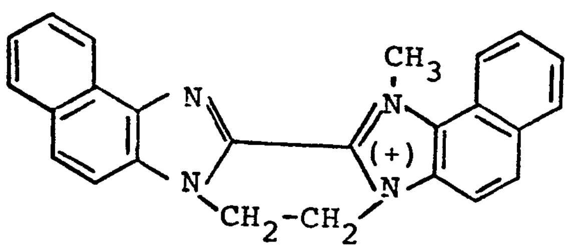

$CH_3OSO^{(-)}$ (106)

werden 7,2 g 3,3'-Dimethylen-2,2'-binaphthimidazol in 300 ml Dimethylformamid suspendiert. Nach Zugabe von 2 ml Dimethylsulfat wird 2 Stunden bei 80°C gerührt, wobei eine klare Lösung entsteht. Dann kühlt man auf 0°C, filtriert und wäscht den Filterkuchen mit Toluol. Nach dem Trocknen im Vakuum erhält man 5,6 g der gewünschten Verbindung, die aus Dimethylformamid umkristallisiert werden kann. Die Verbindung beginnt bei 348°C zu sintern und zersetzt sich ab 383°C. Gelöst in Dimethylformamid zeigt die Verbindung eine kräftige blaue Fluoreszenz.

Das als Ausgangsprodukt verwendete 1,1'-Dimethylen-2,2'-bi-naphthimidazol wird wie folgt hergestellt:
6,7 g 2,2'-Bi-naphthimidazol werden in 100 ml Dimethylformamid zusammen mit 7,0 g Kaliumborat und 2,2 ml 1,2-Dibromethan 1 Stunde bei 80°C gerührt. Anschließend kühlt man auf 0°C und filtriert das Reaktionsprodukt ab. Der Filterkuchen wird mit Dimethylformamid, Methanol und Wasser salzfrei gewaschen und anschließend aus Dimethylformamid umkristallisiert. Ausbeute: 3,4 g. Das Rohprodukt zeigt korrekte Elementaranalysewerte und schmilzt höher als 360°C.

Das als Ausgangsprodukt verwendete 2,2'-Bi-naphthimidazol wird wie folgt hergestellt:
58 g 1,2-Diaminonaphthalin-dihydrochlorid, 31,8 g Soda und 13,2 g Oxalsäurediamid werden in 100 ml Glykol suspendiert. Unter Rühren erhitzt man 50 Stunden zum Rückfluß, läßt danach abkühlen, verdünnt mit 100 ml Ethanol und filtriert. Der Filterkuchen wird mit Wasser salzfrei gewaschen und kann durch Auskochen mit Eisessig weiter gereinigt werden. Schmelzpunkt höher als 360°C.

BEISPIEL 7:

Zur Herstellung der quartären Verbindung der Formel

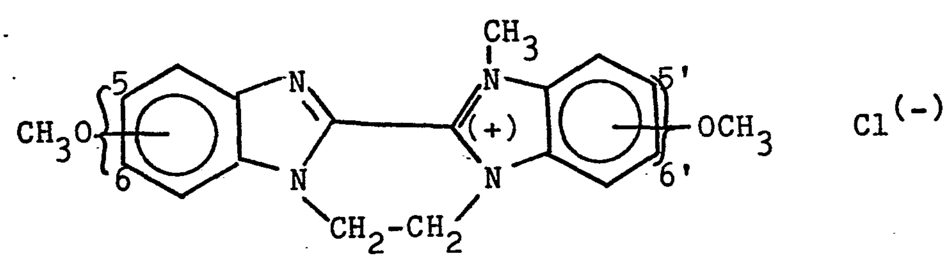

werden 3,2 g 1,1'Dimethylen-2,2'-bi(5 oder 6, 5' oder 6'-dimethoxy)-benzimidazole in 32 ml Dimethylformamid suspendiert und auf 90°C erwärmt. Bei dieser Temperatur wird 1 ml Dimethylsulfat zugegeben und unter Rühren für 3 Stunden auf dieser Temperatur gehalten. Man kühlt auf Raumtemperatur ab, versetzt mit 60 ml Wasser, filtriert und

versetzt das Filtrat mit 10 g Kochsalz. Das ausgefallene Produkt wird nach kurzem Nachrühren abgesaugt und mit gesättigter Kochsalzlösung nachgewaschen und getrocknet. Rohausbeute: 5,45 g, 34 % Kochsalz, entsprechend 97 % der Theorie.

Die Verbindung löst sich in Wasser und Dimethylformamid mit blauer Fluoreszenz.

BEISPIEL 8:

Zur Gewinnung der folgenden Verbindung

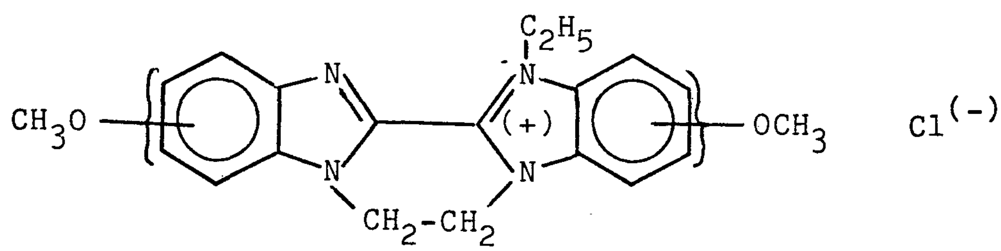

werden 3,2 g 1,1'Dimethylen-2,2'-bi(5 oder 6, 5' oder 6'-dimethoxy)-benzimidazole in 25 ml Dimethylformamid suspendiert, mit 1,3 ml Diethylsulfat versetzt und bei 100°C 6 Stunden verrührt. Anschließend wird mit 50 ml Wasser versetzt, vom ungelösten Rückstand abfiltriert und das Filtrat erkalten gelassen. Man salzt mit 8 g Kochsalz aus, saugt den gebildeten Niederschlag ab und wäscht mit gesättigter Kochsalzlösung nach. Nach dem Trocknen werden 4,5 g eines schwach gelben Pulvers mit einem Kochsalzgehalt von 32,3 % erhalten. Rohausbeute: 79,2 % der Theorie.

Die Verbindung löst sich in Wasser und Dimethylformamid mit grünstichig-blauer Fluoreszenz.

Das als Ausgangsprodukt benötigte 1,1'-Dimethylen-2,2'-bi-(5 oder 6, 5' oder 6'-dimethoxy)-benzimidazol wird wie folgt erhalten:

14,7 g 2,2'-Bi(5 oder 6, 5' oder 6'-dimethoxy)-benzimidazol werden zusammen mit 16,6 g Kaliumcarbonat in 100 ml Dimethylformamid suspendiert. Nach Zugabe von 5,5 ml 1,2-Dibromethan wird unter Stickstoff 12 Stunden bei 100°C gerührt. Nach dem Abkühlen auf Raumtemperatur wird der ausgefallene Niederschlag abgesaugt und das Nutschgut mit Wasser neutral gewaschen.
Rohausbeute: 9,25 g, entsprechend 57,7 % der Theorie.
Das über das Chlorhydrat gereinigte Produkt zeigt einen Schmelzpunkt von 319 bis 322°C.

Zur Herstellung des 2,2'-Bi(5 oder 6, 5' oder 6'-dimethoxy)-benzimidazols werden 211 g des Dichlorhydrats des 3,4-Diaminoanisols in 600 ml Glykol mit 106 g Soda versetzt und nach Zugabe von 44 g Oxalsäurediamid 72 Stunden zum Sieden erhitzt. Das dunkle Reaktionsgemisch wird auf Raumtemperatur abgekühlt, abgesaugt, mit 300 ml Methanol und mit viel heißem Wasser gewaschen.
Rohausbeute: 80 g, entsprechend 54,4 % der Theorie.
Nach mehrfachem Umlösen aus Eisessig und Trichlorbenzol zeigt das Produkt einen Schmelzpunkt von 322 bis 324°C.

BEISPIEL 9:

Die folgende Verbindung

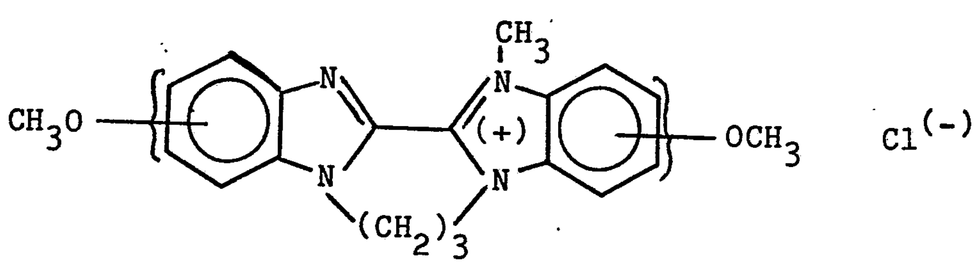

wird hergestellt durch Verrühren von 2,5 g 1,1'Trimethylen-2,2'-bi(5 oder 6, 5' oder 6'-dimethoxy)-benzimidazol in 30 ml Dimethylformamid mit 0,75 ml Dimethylsulfat für 8 Stunden bei 100°C. Das Reaktionsgemisch wird nach dem Erkalten mit

50 ml Wasser verdünnt, filtriert und mit 7 g Kochsalz ausgesalzen. Das ausgefallene Produkt wird isoliert und mit gesättigter Kochsalzlösung gewaschen.
Rohausbeute: 2,9 g, 30 % Kochsalz, entsprechend 70,5 % der Theorie.

Die Verbindung zeigt in Wasser und Dimethylformamid eine blaue Fluoreszenz im Tageslicht.

Das als Ausgangsmaterial benötigte 1,1'-Trimethylen-2,2'-bi(5 oder 6, 5' oder 6'-dimethoxy)-benzimidazol wird auf folgendem Wege erhalten:

14,7 g 2,2'-Bi-(5 oder 6, 5' oder 6'-dimethoxy)-benzimidazol werden in 100 ml Dimethylformamid suspendiert und nach Zugabe von 16,6 g Kaliumcarbonat und 7,6 ml 1,3-Dibrompropan unter Stickstoff 6 Stunden bei 100°C verrührt. Anschließend wird auf Raumtemperatur abgekühlt, abgesaugt und das Filtrat mit 150 ml 2n Salzsäure versetzt. Man erhitzt auf Siedetemperatur und läßt das Chlorhydrat unter Abkühlung auskristallisieren. Durch nochmaliges Auflösen in Wasser und Zugabe von Sodalösung bis zur schwach alkoholischen Reaktion wird die freie, überbrückte Bis-benzimidazolverbindung erhalten.
Ausbeute: 5,1 g, entsprechend 30,5 % der Theorie.
Schmelzpunkt: 282 - 285°C.

BEISPIEL 10:

Zur Herstellung der unsymmetrisch substituierten Verbindung

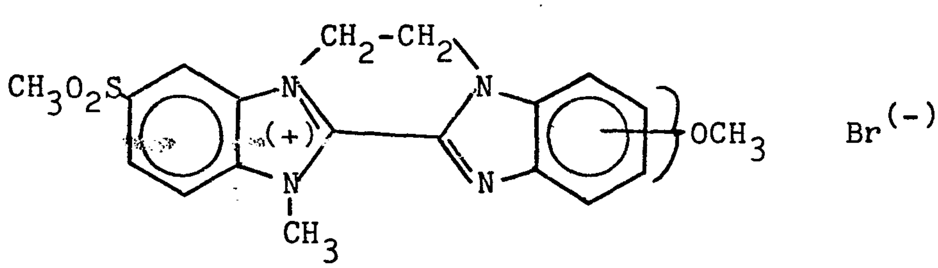

werden 1,07 g 1-Methyl-2,2'-bi(5 methylsulfonyl, 5' oder 6'-methoxy)-benzimidazol in einer Mischung aus 20 ml Dimethylformamid und 10 ml 1,2-Dibromäthan unter Zusatz von 0,6 g Kaliumcarbonat 10 Stunden auf 100°C erhitzt. Der gebildete Niederschlag wurde bei Raumtemperatur abgesaugt und mit Aceton gewaschen. Nach Umlösung aus Wasser werden 0,59 g eines gelben Pulvers vom Schmelzpunkt 263-266°C erhalten, entsprechend einer Ausbeute von 42,5 % der Theorie.

Gelöst in Dimethylformamid zeigt diese Verbindung im Tageslicht eine kräftige blaue Fluoreszenz.

Das als Ausgangsprodukt benötigte 1-Methyl-2,2'-bi(5-methylsulfonyl, 5' oder 6'-methoxy)-benzimidazol wird wie folgt hergestellt:

10,17 g N-Methyl-5-methylsulfonyl-benzimidazol-2-carbonsäure werden unter Kühlung in 50 ml Phosphoroxychlorid suspendiert, mit 9,16 g Phosphorpentachlorid versetzt und auf Siedetemperatur angeheizt. Unter Rührung wird 4 1/2 Stunden am Rückfluß gekocht und anschließend Phosphoroxychlorid im Vakuum abdestilliert. Der Rückstand wird mit 300 ml Chlorbenzol verrührt, abgesaugt und mit 35 ml Chlorbenzol gewaschen. Das so erhaltene Säurechlorid wird in 30 ml Chlorbenzol suspendiert, mit 8,4 g 3-Nitro-4-amino-anisol und unter Rührung tropfenweise mit 12,8 g N,N-Dimethylanilin versetzt. Anschließend wird auf 60°C geheizt und 3 Stunden bei dieser Temperatur verrührt. Man läßt erkalten, saugt ab und wäscht mit Methanol und Wasser frei von Chlorionen. Nach Trocknung werden 11,8 g der Acylaminoverbindung

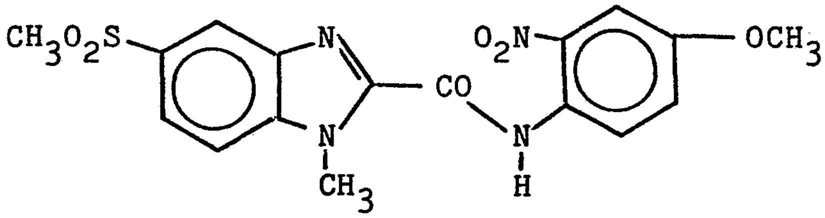

vom Schmelzpunkt 253-257°C erhalten, entsprechend 73 % der Theorie.

11,23 g dieser Acylaminoverbindung werden in 150 ml Dimethylformamid in Gegenwart von 1,5 g Raney-Nickel bei einem Druck von 100 bar bei Raumtemperatur hydriert. Nach dem Abfiltrieren des Katalysators wird das Lösungsmittel abgedampft und der Rückstand durch Behandlung mit Methanol zur Kristallisation gebracht.

Ausbeute: 7,96 g der Verbindung der folgenden Formel vom Schmelzpunkt 234-241°C, entsprechend 77 % der Theorie.

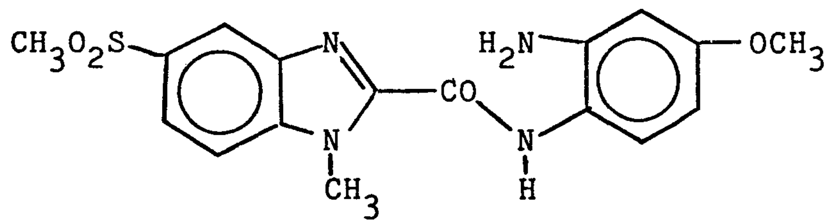

Zum Benzimidazolringschluß werden 7,96 g dieser Verbindung in 40 ml Eisessig 2 1/2 Stunden am Rückfluß gekocht. Das Reaktionsgemisch wird mit 40 ml Wasser verdünnt, bei Raumtemperatur abgesaugt und mit Wasser gewaschen.

Rohausbeute: 6,65 g 1-Methyl-2,2'-bi(5-methylsulfonyl, 5' oder 6'-methoxy)-benzimidazol, entsprechend 83,6 % der Theorie. Nach Umlösung aus o-Dichlorbenzol zeigt die Verbindung der Formel

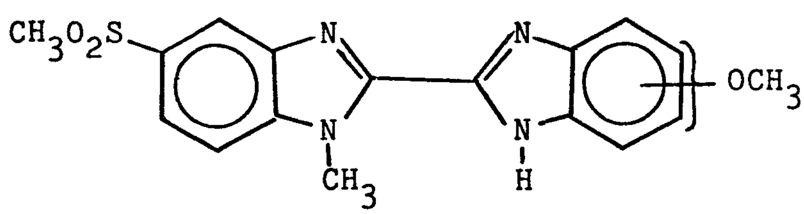

einen Schmelzpunkt von 282-284°C.

BEISPIEL 11:

Zur Herstellung der unsymmetrisch substituierten Verbindung

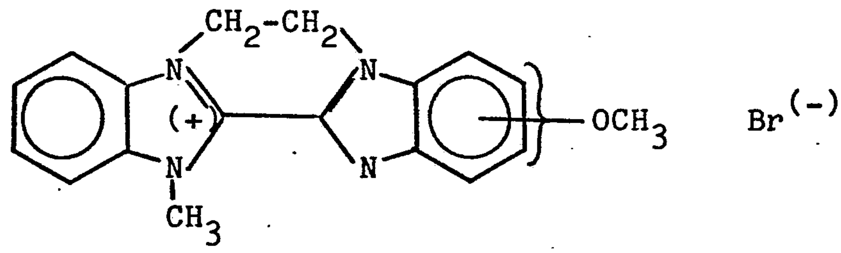

werden 1,39 g 1-Methyl-2,2'-bi(5' oder 6'-methoxy)-benzimidazol in 15 ml Dimethylformamid gelöst und mit 0,9 ml 1,2-Dibromäthan 0,7 g Kaliumcarbonat versetzt. Man rührt 6 Stunden bei 100°C, kühlt auf Raumtemperatur ab und verdünnt mit 50 ml Toluol. Das ausgefallene Reaktionsprodukt wird abgesaugt und mit Toluol gewaschen. Nach Umlösung aus 70 ml Wasser unter Zugabe von Kohle werden 1,21 g eines gelben Pulvers vom Schmelzpunkt 278-285°C u. Zers. erhalten, entsprechend einer Ausbeute von 63 % der Theorie.

Gelöst in Dimethylformamid zeigt die so erhaltene Verbindung im Tageslicht eine grünstichig-blaue Fluoreszenzfarbe.

Das als Ausgangsprodukt benötigte 1-Methyl-2,2'-bi(5' oder 6'-methoxy)-benzimidazol wird auf folgendem Weg erhalten:

14,0 g N-Methyl-benzimidazol-2-carbonsäure werden unter leichter Kühlung bei einer Temperatur unter 30°C in 100 ml Phosphoroxychlorid eingetragen, mit 18,3 g Phosphorpentachlorid versetzt und 20 Stunden bei Raumtemperatur verrührt. Bei 40-50°C wird im Vakuum abgezogen, der Rückstand mit 60 ml Toluol verrührt, abgesaugt und mit Toluol gewaschen. Das so erhaltene Säurechlorid wird in 70 ml Toluol eingetragen, mit 16,8 g 3-Nitro-4-amino-anisol und unter Kühlung (230°C) mit 25,6 g N,N-Dimethylanilin tropfenweise versetzt. Man verrührt anschließend 6 Stunden auf 60°C, zieht das Lösungsmittel im Vakuum ab, verrührt den Rückstand mit Methanol und saugt ab. Es wird mit Methanol und Wasser gewaschen und bei 60°C im Vakuum getrocknet. Es werden 20,3 g der Acylaminoverbindung

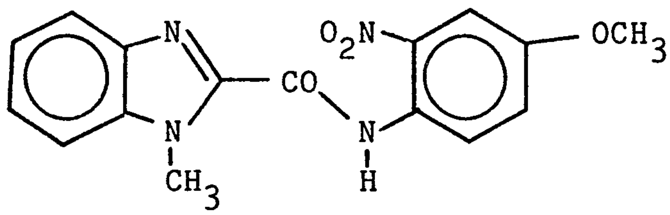

vom Schmelzpunkt 209-211°C erhalten, entsprechend 77 % der Theorie.

Die katalytische Reduktion dieser Acylaminoverbindung erfolgt in der im Beispiel 10 angegebenen Weise. Ausbeute: 87 % der Theorie, Schmp. 186 - 189°C.

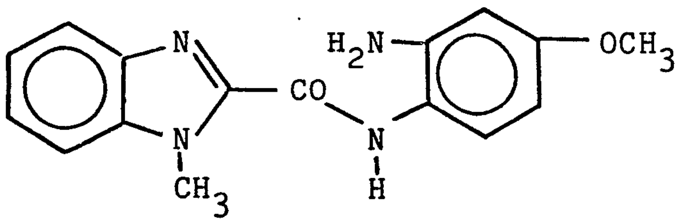

Auch der Benzimidazolringschluß wurde nach der im Beispiel 10 angegebenen Arbeitsweise durchgeführt. In 96 %-iger Rohausbeute wurde das 1-Methyl-2,2'-bi(5' oder 6'-methoxy)-benzimidazol erhalten, das nach Umlösung aus Toluol einen Schmelzpunkt von 245-247°C zeigte.

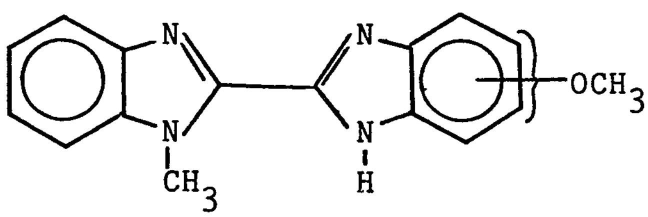

## BEISPIEL 12:

Die Verbindung

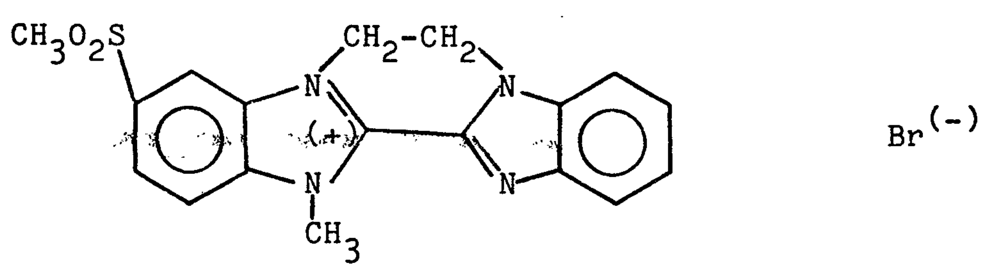

vom Schmelzpunkt 326°C u. Zers., kann analog zu den vorherigen Beispielen aus den folgenden Vorstufen hergestellt werden:

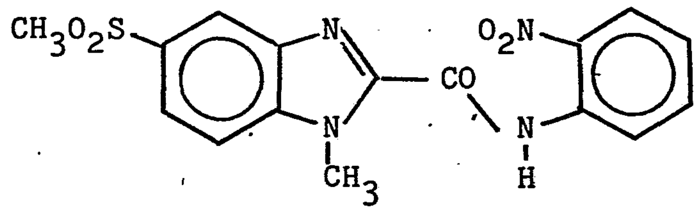

Grünliches Pulver vom Schmelzpunkt 250 - 251°C u. Zers.

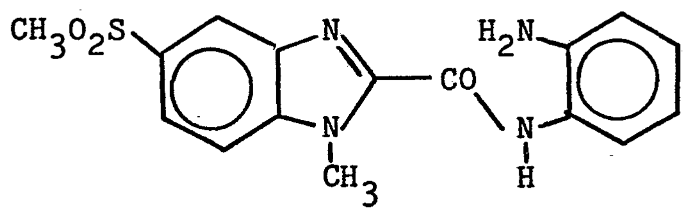

Olivfarbenes Pulver vom Schmelzpunkt 224 - 225°C u. Zers.

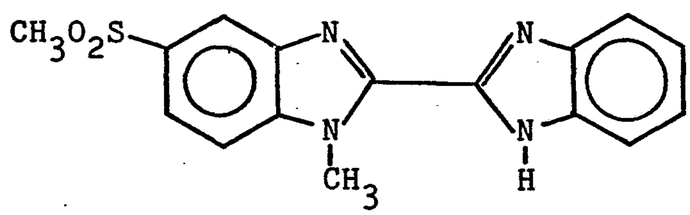

Hellgelbe Verbindung, Schmelzpunkt 296,5-298,5°C (aus o-Dichlorbenzol)

BEISPIEL 13:

In 200 ml Wasser werden 0,24 g Natriumchlorit, 0,1 g eines handelsüblichen Bleichhilfsmittels und 0,3 ml 2n Essigsäure bei 60°C gelöst. Vom optischen Aufheller der Formel (101) wird eine Lösung hergestellt, in dem man 0,02 g in 1 ml DMF löst. Man gießt diese Lösung in das wäßrige Bad und stellt durch Zutropfen von Essigsäure (10%ig) den pH-Wert auf 3,5. In diese wäßrige, den Aufheller enthaltende Flotte gibt man bei 60°C ein 5 g schweres Polyacrylnitrilgewebe. Man bleicht

zuerst eine halbe Stunde bei 80°C vor und färbt noch eine halbe Stunde bei 100°C nach. Es wird heiß und kalt mit Wasser gespült und getrocknet. Das so behandelte Fasermaterial zeigt ein gefälliges, weißes Aussehen mit einer violetten Nuance. Eine ähnliche Wirkung erreicht man, wenn man in Abwesenheit von Natriumchlorit und des Bleichhilfsmittels arbeitet und den pH-Wert der Flotte mit Essigsäure (10%ig) auf 4 stellt.

Verwendet man anstelle des vorstehend beschriebenen Aufhellers einen Aufheller der Formeln (102), 103, (104) oder (105) und verfährt im übrigen wie im Beispiel angegeben, so erhält man ähnliche Effekte auf dem genannten Fasermaterial.

BEISPIEL 14:

Zu 100 ml Wasser werden 0,12 ml 85%ige Ameisensäure gegeben. Vom optischen Aufheller der Forme (101) wird eine Lösung hergestellt, indem man 1 g in 1000 ml Wasser löst. Von dieser Stammlösung gibt man 1,5 ml zu der oben beschriebenen Lösung. Die so erhaltene Flotte wird auf 60°C erwärmt und in diese ein 3 g schweres Polyacrylnitrilgewebe gegeben. Man steigert die Temperatur innerhalb von 10 bis 15 Minuten auf 95 bis 98°C und beläßt bei dieser Temperatur eine Stunde. Das Gewebe wird sodann 2 Minuten in fliessendem kalten Wasser gespült und anschließend 20 Minuten bei 60°C getrocknet. Das so behandelte Gewebe zeigt ein weißes brillantes Aussehen.

Verfährt man wie im obigen Beispiel angegeben, verwendet aber anstelle des dort genannten Aufhellers die Verbindung der Formeln (102), (103), (104) oder (105), so erhält man ähnliche Resultate.

BEISPIEL 15:

Verfährt man wie bei Beispiel 7, setzt jedoch als Aufheller die Verbindung

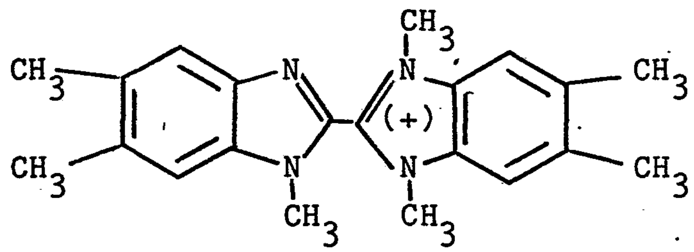

$CH_3OSO_3^{(-)}$

ein, d. h. also die (101) analoge Verbindung ohne Ethylenbrücke, so zeigt das so behandelte Textilgut keinerlei Aufhellung, wodurch gezeigt wird, daß erst die Einführung der Ethylengruppe eine Aufhellwirkung hervorruft.

PATENTANSPRÜCHE:

1. Verbindungen der allgemeinen Formel (1)

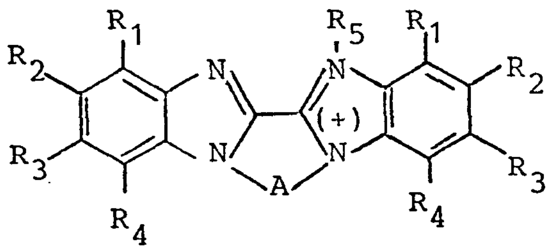

$X^{(-)}$ (1)

wobei $R_1$ Wasserstoff, Halogen, Alkyl, Alkoxy oder zusammen mit $R_2$ einen ankondensierten Benzoring, $R_2$ Wasserstoff, Alkyl, Alkoxy, Halogen, Phenyl, Cyan, Carboalkoxy, Carboxy, Carbonamido, Mono- oder Di-alkylcarbonamido, Sulfo, Alkylsulfonyl, Alkyloxysulfonyl, Sulfonamido, Mono- oder Di-alkylsulfonamido oder zusammen mit $R_3$ einen ankondensierten Benzoring,
$R_3$ die gleiche Bedeutung hat wie $R_2$,
$R_4$ Wasserstoff, Alkyl, Alkoxy, Halogen oder zusammen mit $R_3$ einen ankondensierten Benzoring,
$R_5$ Alkyl, Hydroxyalkyl, Alkoxyalkyl, gegebenenfalls substituiertes Aralkyl, Cyanäthyl, Cycloalkyl oder eine Gruppe der Formeln
$-CH_2CN$, $-CH_2CONH_2$ oder $-CH_2COO$ Alkyl,
A eine Gruppe der Formeln

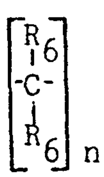 oder $-(CH_2)_4-$

$R_6$ Wasserstoff, Alkyl, Hydroxyalkyl, Alkoxyalkyl, Aralkyl oder gegebenenfalls substituiertes Phenyl,
n 1,2 oder 3 und
$X^{(-)}$ ein Halogenid-, Alkylsulfonat-, Alkylsulfat-, Alkylphenylsulfonat- oder Phenylsulfonat-ion bedeutet.

2. Verbindungen der Formel (1), worin
$R_1$ Wasserstoff, Alkyl oder zusammen mit $R_2$ einen ankondensierten Benzoring,
$R_2$ Wasserstoff, Alkyl, Alkoxy, Halogen, Cyan, Carboalkoxy, Dialkylcarbonamido, Sulfo, Alkylsulfonyl, Alkyloxysulfonyl, Sulfonamido oder zusammen mit $R_3$ einen ankondensierten Benzoring,
$R_3$ die gleiche Bedeutung wie $R_2$ hat,
$R_4$ Wasserstoff, Alkyl oder zusammen mit $R_3$ einen ankondensierten Benzoring,
$R_5$ Alkyl, Hydroxyalkyl, Alkoxyalkyl, gegebenenfalls substituiertes Aralkyl, Cyanäthyl oder eine Gruppe der Formeln
$-CH_2CN$, $-CH_2CONH_2$, $-CH_2COO$ Alkyl,
A eine Gruppe der Formeln

$$\left[-\overset{R_6}{\underset{R_6}{C}}-\right]_n \quad \text{oder} \quad -(CH_2)_4-$$

$R_6$ Wasserstoff, Alkyl, Hydroxyalkyl, Alkoxyalkyl, Aralkyl oder gegebenenfalls substituiertes Phenyl,
n 1, 2 oder 3 und $X^{(-)}$ ein Halogenid-, Alkylsulfonat-, Alkylsulfat-, Alkylphenylsulfonat- oder Phenylsulfonat-ion bedeutet.

3. Verbindungen der Formel (1),
worin $R_1$ und $R_4$ Wasserstoff, Methyl, Ethyl, Methoxy, Chlor oder $R_1$ zusammen mit $R_2$ bzw. $R_3$ zusammen mit $R_4$ einen ankondensierten Benzoring,
$R_2$ und $R_3$ Wasserstoff, Methyl, Ethyl, Chlor, Alkylsulfonyl, Cyan, Carboxy, Carboalkoxy oder Carbonamido,
$R_5$ Alkyl, Hydroxyalkyl, gegebenenfalls durch Chlor, Methyl oder Methoxy substituiertes Benzyl oder eine Gruppe der Formeln
$-CH_2CN$, $-CH_2CONH_2$, oder $-CH_2COO$ Alkyl,

A Methylen, Ethylen, Trimethylen oder Tetramethylen, die jeweils durch Alkyl, Alkoxy, Phenyl, Cyanphenyl oder Chlorphenyl substituiert sein können und $X^{(-)}$ ein Chlorid-, Ethosulfat-, Methosulfat-, Methylsulfonat-, Tolylsulfonat- oder Phenylsulfonat-ion bedeutet.

4. Verbindungen der Formel 1, worin $R_1$ und $R_4$ Wasserstoff, $R_2$ Wasserstoff, Alkyl, Alkoxy oder Chlor, $R_3$ Wasserstoff, Alkyl, Alkoxy, Chlor, Methylsulfonyl, Cyan oder Carboalkoxy, $R_5$ Methyl, A Methylen, Ethylen oder Propylen und X Chlorid, Methosulfat, Ethosulfat, Methylsulfonat oder p-Tolylsulfonat bedeutet.
Besonders bevorzugt sind auch die Verbindungen der Formel (1), worin $R_1$ und $R_4$ Wasserstoff, $R_2$ Wasserstoff, Alkyl, Alkoxy oder Chlor, $R_3$ Wasserstoff, wenn $R_2$ ungleich Wasserstoff ist, sowie Alkyl, Chlor, Methoxy, Ethoxy, Methylsulfonyl, Cyano, Carboalkoxy, $R_5$ Methyl, A Ethylen und $X^{(-)}$ Chlorid, Methosulfat, Methylsulfonat oder Tolylsulfonat bedeutet.

5. Verfahren zur Herstellung der Verbindungen der Formel (1) dadurch gekennzeichnet, daß man eine Verbindung der Formel (2)

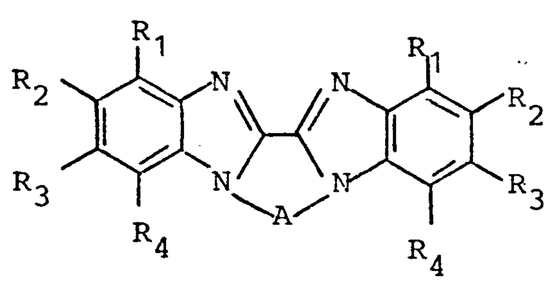

mit einer Verbindung der Formel

$$X - R_5$$

alkyliert.

6. Verwendung der Verbindungen der Formel (1) als optische Aufheller.

Europäisches Patentamt

# EUROPÄISCHER RECHERCHENBERICHT

0027897 Nummer der Anmeldung

EP 80 10 5767.0

| EINSCHLÄGIGE DOKUMENTE | | | KLASSIFIKATION DER ANMELDUNG (Int. Cl.3) |
|---|---|---|---|
| Kategorie | Kennzeichnung des Dokuments mit Angabe, soweit erforderlich, der maßgeblichen Teile | betrifft Anspruch | |
| | ZEITSCHRIFT FÜR NATURFORSCHUNG, Band 25b, Nr. 7, Juli 1970<br>Tübingen<br>H. RÖCHLING "Quaternäre Diimidazole und Dibenzimidazole"<br>Seiten 931 bis 934<br>* Seite 932, Spalte 2 *<br>-- | 5 | C 07 D 487/14<br>D 06 L 3/12<br>//(C 07 D 487/14, 243/00, 235/00, 235/00)<br>( C 07 D 487/14, 241/00, 235/00, 235/00) |
| | DE - A1 - 2 733 439 (SANDOZ-PATENT-GMBH)<br>* Anspruch 13 *<br>-- | 6 | RECHERCHIERTE SACHGEBIETE (Int. Cl.3) |
| A | TEXTILVEREDLUNG, Band 11, Nr. 9, 1976<br>R. ANLIKER et al. "Das Aufhellen von Polyacrylnitril-Fasern"<br>* Seiten 369 bis 375<br>---- | | C 07 D 487/14<br>D 06 L 3/12 |

KATEGORIE DER GENANNTEN DOKUMENTE
- X: von besonderer Bedeutung
- A: technologischer Hintergrund
- O: nichtschriftliche Offenbarung
- P: Zwischenliteratur
- T: der Erfindung zugrunde liegende Theorien oder Grundsätze
- E: kollidierende Anmeldung
- D: in der Anmeldung angeführtes Dokument
- L: aus andern Gründen angeführtes Dokument
- &: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 29-01-1981 | FROELICH |